# EUROPEAN PATENT APPLICATION

(11) **EP 1 632 489 A1**
(43) Date of publication of application: **08.03.2006**
(21) Application number: 04077400.2
(22) Date of filing: 24.08.2004
(51) Int. Cl.: C07D 403/04, C07D 403/14, A61K 31/515, A61P 29/00, A61P 35/00, A61P 37/00

(54) **5-(1,1'-Biphenyl)-4-yl-5-(4-(4-aminoacylphenyl)-piperazin)-1-yl-pyrimidine-2,4,6-trione derivatives, as inhibitors of zinc metallondopeptidases, their preparation and use.**

(71) Applicant: University of Liege, 4020 Liège (BE)
(72) Inventor: Detry, Vanessa, 6687 Bertogne (BE); Pirotte, Bernard, 4680 Oupeye (BE); Counerotte, Stéphane, 6941 Durbuy (BE); Frankenne, Francis, 4050 Chaudfontaine (BE); Noel, Agnés, 4653 Bolland (BE); Foidart, Jean-Michel, 4870 Trooz (BE)

(57) **Abstract**

New Pyrimidinetrione derivatives represented by formula I 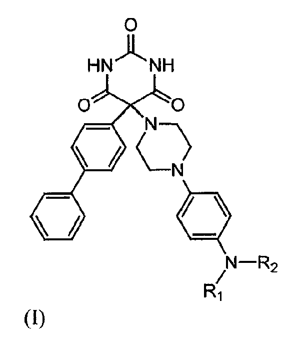 wherein R1 and R2 are defined in the description, composition thereof, and methods of preparation are described.

The compounds are useful in the treatment of disease involving metalloproteinases

## Description

The present invention relates to new pyrimidinetrione derivatives, to their method of preparation, to compositions comprising the compounds, to the compounds for use as medicament and their use in therapy e.g. in preparation of medicament for the treatment of inflammation, cancer and other disorders.

### BACKGROUND OF THE INVENTION

Pyrimidinetrione derivatives are inhibitors of zinc metalloendopeptidases, especially those belonging to the class of matrix metalloproteinases (MMP).

Matrix metalloproteinases (MMPs) are a family of about 24 homologous proteins sharing the capacity to cleave peptidic bounds of many of the structural proteins of the extra-cellular matrix.
They have a structure-conserved active site in which a zinc atom plays an essential role.
The minimum structure configuration of MMPs is a catalytic domain and a pro peptide which is hiding the catalytic site in the inactivated, pro-forms of the enzymes. Activation is obtained by catalytic cleavage of the pro-domain.
Most of the MMPs have an additional hemopexin domain linked to the C-terminal end of the catalytic domain through a so-called hinge peptide. This hemopexin (PEX) domain has the ability to interact with numerous proteic/sugar substrates, which affects the net activity of the enzyme. All the MMPs are secreted in the interstitial medium, but 6 MMPs are anchored to the cell membrane (MT1-6-MMP).
MMPs are classified either following their structure or based on their substrate specificity. The following sub-families have thus been defined: collagenases (MMP1, 8, 13, 18), stromelysins (MMP 3, 10, 11), metalloelastase (MMP12), gelatinases (MMP2, 9), MT-MMPs (MMP 14, 15, 16, 24, 25).

The MMPs are known for their role in numerous physiological processes such as wound healing, ovulation, endometrial cycle, embryo development, trophoblat implantation and bone and cartilage remodelling.
They are also playing a major role in many pathologies, i.e. arthritis, tumour growth, progression and invasion, osteoporosis, ocular abnormal angiogenesis, multiple sclerosis, asthma, atherosclerosis, corneal ulceration, periodontal disease and the like.

Pyrimidinetrione derivatives are already known in the art. WO 01/25217 described pyrimidine-2,4,6 trione derivatives as inhibitors of matrix metalloproteinases but such compounds have in general a low solubility in water and therefore a bad oral biodisponibility . Toxicity by photosensitization of some of them is wellknown.

We have now found new pyrimidinetrione derivatives with improved activity as matrix metallo-proteinase inhibitors over the compounds described in WO01/25217, putatively lower toxicity by photosensitization and better solubility in water.

### DESCRIPTION OF THE INVENTION

The present invention concerns new pyrimidinetriones of general formula I: 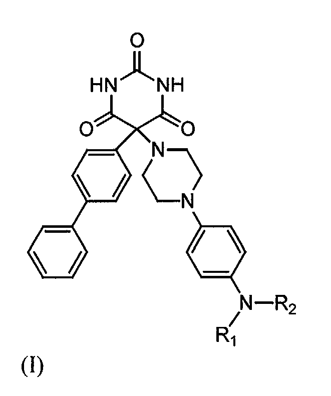
wherein
R₁ is hydrogen; or R₁ forms with R₂ and the nitrogen atom a succinimide (pyrrolidinedione) cycle, a glutarimide (piperidinedione) cycle or a perhydroazepinedione cycle.
R₂ is formyl, a straight or branched C₁-₆-acyl, a straight or branched carboxy-C₁₋₆-alkylcarbonyl, a straight or branched C₁-₆-alkoxycarbonyl, a straight or branched C₁-₆-alkylaminocarbonyl;
   or R₂ forms with R₁ and the nitrogen atom a succinimide (pyrrolidinedione) cycle, a glutarimide (piperidinedione) cycle or a perhydroazepinedione cycle.

The present invention also encompasses
a pharmaceutical acceptable salt thereof with a pharmaceutically acceptable acid or base; an optical isomer thereof, a tautomeric form thereof, or a polymorphic form thereof.

The salts include pharmaceutically acceptable acid addition salts, pharmaceutically acceptable metal salts, such as hydrochloric, hydrobromic, hydroiodic, phosphoric, sulfuric, trifluoroacetic, oxalic, maleic, pyruvic, malonic, succinic, citric, tartaric, fumaric, mandelic, benzoic, cinnamic, methanesulfonic, ethane sulfonic, picric and the like and include acids related to the pharmaceutically acceptable salts listed in Journal of Pharmaceutical Sciences 66, 2 (1977) and incorporated herein by reference, or lithium, sodium, potassium, magnesium and the like.

The term "C₁-₆-alkyl" as used herein, alone or in combination, refers to a straight or branched hydrocarbon chain having 1-6 carbon atoms such as e.g. methyl, ethyl, propyl, isopropyl, butyl, tert-butyl, pentyl, hexyl, isobutyl, 1,2-dimethylpropyl and the like.

The term "C₁₋₆-acyl" as used herein refers to a monovalent substituent comprising a C₁₋₆-alkyl group linked through a carbonyl group; such as e.g. acetyl, propionyl, butyryl, isobutyryl, pivaloyl, valeryl, and the like.

The term "carboxy-C₁₋₆-alkylcarbonyl" as used herein refers to a monovalent substituent comprising a carboxy group (-COOH) linked through a C₁₋₆-alkyl group which in turn is linked through a carbonyl group; such as carboxyethylcarbonyl (or succinyl), carboxypropylcarbonyl (or glutaryl), carboxybutylcarbonyl, and the like.

The term "C₁₋₆-alkoxycarbonyl" as used herein refers to a monovalent substituent comprising a C₁₋₆-alkoxy group linked through a carbonyl group; such as e.g. methoxycarbonyl, ethoxycarbonyl, propoxycarbonyl, sec-butoxycarbonyl, tert-butoxycarbonyl and the like.

The term "C₁₋₆-alkylaminocarbonyl" as used herein refers to a monovalent substituent comprising a C₁₋₆-alkyl group linked through an aminocarbonyl group; such as e.g. methylaminocarbonyl, ethylaminocarbonyl, propylaminocarbonyl, sec-butylaminocarbonyl, tert-butylaminocarbonyl and the like.

The term "pyrrolidinedione" as used herein refers to a saturated five-membered ring comprising one nitrogen atom and bearing two carbonyl groups in each ortho position of the nitrogen atom.

The term "piperidinedione" as used herein refers to a saturated six-membered ring comprising one nitrogen atom and bearing two carbonyl groups in each ortho position of the nitrogen atom.

The term "perhydroazepinedione" as used herein refers to a saturated seven-membered ring comprising one nitrogen atom and bearing two carbonyl groups in each ortho position of the nitrogen atom.

Preferred compounds of the invention are selected from the group consisting of:
*N*-{4-[4-(5-(1,1'-biphenyl)-4-yl-2,4,6-trioxoperhydropyrimidin-5-yl)piperazin-1-yl]-phenyl}succinamic acid;
*N*-{4-[4-(5-(1,1-Biphenyl)-4-yl-2,4,6-trioxoperhydropyrimidin-5-yl)piperazin-1-yl]-phenylcarbamoyl}butyric acid;
5-(1,1'-Biphenyl)-4-yl-5-{4-[4-(2,5-dioxopyrrolidin-1-yl)phenyl]piperazin-1-yl}pyrimidine-2,4,6(1*H*,3*H*,5*H*)-trione;
5-(1,1'-Biphenyl)-4-yl-5-{4-[4-(2,5-dioxopiperidin-1-yl)phenyl]piperazin-1-yl}pyrimidine-2,4,6(1*H*,3*H*,5*H*)-trione;
*N*-{4-[4-(5-(1,1'-Biphenyl)-4-yl-2,4,6-trioxoperhydropyrimidin-5-yl)piperazin-1-yl]-phenyl}acetamide;
*N*-{4-[4-(5-(1,1'Biphenyl)-4-yl-2,4,6-trioxoperhydropyrimidin-5-yl)-piperazin-1-yl]phenyl}-formamide.

The compounds of the present invention inhibit metalloproteinases which make them useful in the treatment of various diseases. Indeed, MMPs are playing a major role in many pathologies, i.e. arthritis, tumour growth, progression and invasion, osteoporosis, ocular abnormal angiogenesis, multiple sclerosis, asthma, atherosclerosis and the like.

Accordingly, in another aspect the invention relates to a compound of the general formula I or a pharmaceutically acceptable salt thereof for therapeutical use, particularly for therapeutical use in the treatment of diseases involving metalloproteinases such as arthritis, tumour growth, progression and invasion, osteoporosis, ocular abnormal angiogenesis, multiple sclerosis, asthma, atherosclerosis and the like.

The invention also relates to the use of a compound of the general formula I, a pharmaceutical acceptable salt thereof with a pharmaceutically acceptable acid or base, an optical isomer thereof, a tautomeric form thereof, or a polymorphic form thereof for preparing a medicament, particularly for the treatment of diseases involving metalloproteinases.

The treatment of diseases involving metalloproteinases comprises administering to a patient an amount of one or more compounds of the invention. As used herein, the term treatment is intended to refer to prevention, amelioration, or reduction in severity of a symptom involving metalloproteinases.
The compounds of the invention may be administered singly or in combination. Typically the compounds of the invention are administered as a dose in an amount of 0.05 mg to 1000mg per day, preferably from 0.1 mg to 500mg per day and most preferably from 0.1mg to 200mg per day. However, other amounts, including substantially lower or higher amounts, may also be administered. The compounds of the invention may be administered to a human subject intramuscularly, subcutaneously, intravenously or by any other route of administration.

In yet another aspect, the present invention also relates to methods of preparing the compounds of the present invention.

A first method comprises a step of :
a) reacting a compound of formula II: 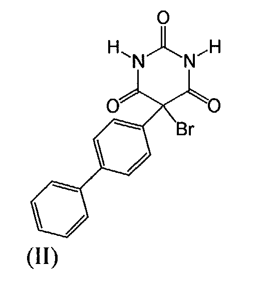 which is obtained as described by Daniewski et al., Organic Research & Development 2004, 8, 411-414 and incorporated herein by reference, with a compound of formula III: 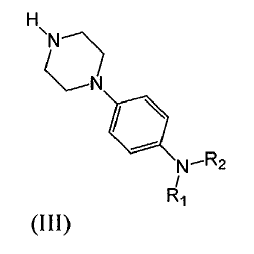 wherein R₁ and R₂ are defined as above, to form a compound of the general formula I.
   The compound of formula III can be obtained as described in scheme 1, wherein tert-butyl 4-(4-aminophenyl)piperazine-1-carboxylate (IV) obtained as described by Koshio et al., Bioorg. Med. Chem. 2004, 12, 2179-2191 and incorporated herein by reference, is reacted in step i with an appropriate anhydride ((R'CO)₂O or HCOOCOCH₃), chloroformiate (R'OCOCl) or isocyanate (R'NCO) wherein R' is a straight or branched C₁₋₆ alkyl chain to give an intermediate of general formula V. When succinic anhydride or glutaric anhydride is used, ring closure occurred by using carbonyldiimidazole in presence of triethylamine. Deprotection in step ii by trifluoroacetic acid afforded compound of the general formula III. 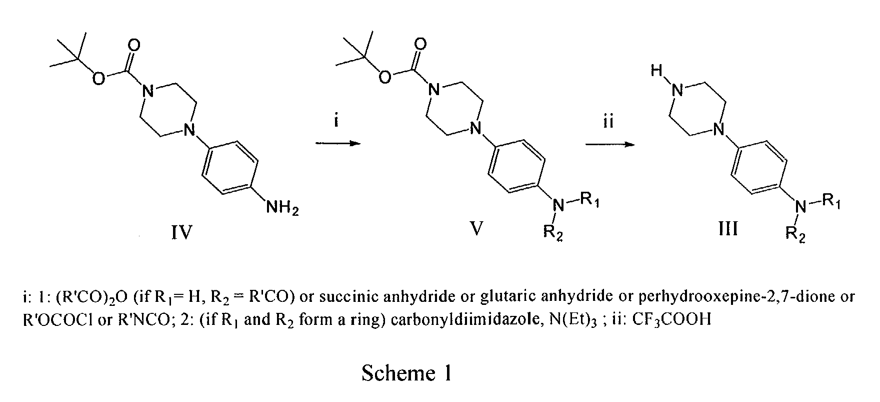
   A second method comprises a step of:
b) reacting a compound of formula VI: 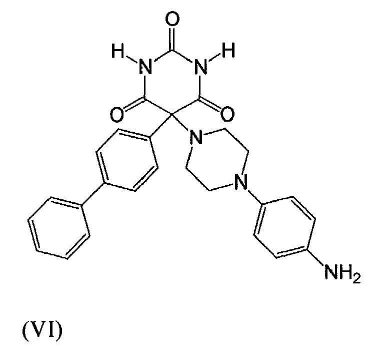
   which is obtained from reduction of the corresponding nitro derivative (synthesized as described by Daniewski et al. in Organic Research & Development 2004, 8, 411-414) by catalytic hydrogenation, with an appropriate chloroformiate (R'OCOCl), isocyanate (R'NCO) or anhydride selected from the group consisting of (R'CO)₂O, HCOOCOCH₃, succinic anhydride, glutaric anhydride or perhydrooxepine-2,7-dione, wherein R' is a straight or branched C₁₋₆ alkyl chain, optionally followed by a ring closure, as reported on scheme 1 (step i.2), to form a compound of the general formula I.
   A third method of preparing the compounds according to the invention comprises a step of
c) reacting a compound of formula II, which is obtained as described by Daniewski et al. in Organic Research & Development 2004, 8, 411-414 and incorporated herein by reference 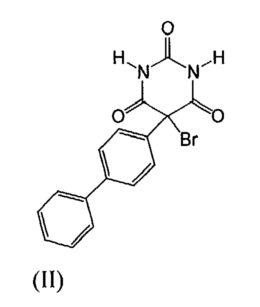 with a compound such as VII, commercially available 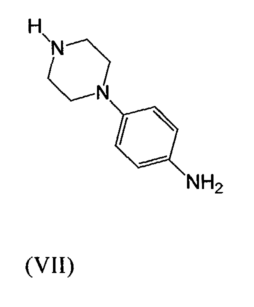 to obtain the compound of formula VI, which is reacted with an appropriate chloroformiate (R'OCOCI), isocyanate (R'NCO) or anhydride selected from the group consisting of (R'CO)₂O, HCOOCOCH₃, succinic anhydride, glutaric anhydride or perhydrooxepine-2,7-dione, wherein R' is a straight or branched C₁₋₆ alkyl chain, optionally followed by a ring closure, as reported on scheme 1 (step i.2), to form a compound of the general formula I.

### PHARMACOLOGICAL RESULTS

The matrix metalloproteinase (MMP) inhibitory activity has been evaluated in a classical biochemical assay using fluorigenic peptide substrates. Briefly, a peptide bearing 1) a fluorescent group and 2) a quenching group bound to another amino acid of the peptide is submitted to the proteolytic action of an MMP. The cleavage of peptide bounds due to the MMP results in a dequenching of the fluorescent group. There is a direct relationship between the observed fluorescence and the activity of the enzyme. The MMP inhibiting activity of a given substance can then be assessed using this assay.
The compound of the present invention has been assayed using this assay design with recombinant human MMP2, MMP14 and MMP17 and the peptide ZGly-Gly-ArgAMC, which is a well-known, commercially available substrate of MMPs.
The following IC50 values (inhibitor concentration required to inhibit 50% of the enzyme) have been obtained with the compound of example 4: 75 nM (nanomolar) for MMP2, 13 nM for MT1-MMP (MMP14) and 21 nM for MT3-MMT (MMP17).

### EXAMPLES

The method of preparation the compounds of formula I is further illustrated in the following examples which, however are not to be construed as limiting.

### Example 1: preparation of

### 5-[4-(4-Aminophenyl)piperazin-1-yl]-5-[1,1'-biphenyl]-4-ylpyrimidine-2,4,6(1H,3H,5H)-trione

5-[1,1'-Biphenyl]-4-yl-5-[4-(4-nitrophenyl)-1-piperazin-1-yl]-pyrimidine-2,4,6(1*H*,3*H*,5*H*)-trione (500 mg) was dissolved in hot ethanol (100 ml). 10% Pd/C (50 mg) was added to the solution. The mixture was placed in a Paar apparatus for 2 hours under 4 bars hydrogen pressure at 50°C. After hydrogenation, ethanol was removed under reduced pressure. The residue was dissolved in acetone (250 ml). The 10% Pd/C was removed by filtration over a double 602H filter. 5-[1,1'-biphenyl]- 4-yl-5-[4-(4-aminophenyl)-1-piperazinyl]-pyrimidine-2,4,6(1*H*,3*H*,5*H*)-trione was precipitated by addition of water. The precipitate was collected by filtration. The title compound was dried in a vacuum system (containing NaOH pellets) at room temperature: melting point : 268-269 °C.

### Example 2: preparation of

### N-{4-[4-(5-(1,1'-biphenyl)-4-yl-2,4,6-trioxoperhydropyrimidin-5-yl)piperazin-1-yl]-phenyl}succinamic acid

Succinic anhydride (200 mg) was dissolved in dimethylformamide (5 ml). 5-[4-(4-Aminophenyl)piperazin-1-yl]-5-[1,1'-biphenyl]-4-ylpyrimidine-2,4,6(1*H*,3*H*,5*H*)-trione (500 mg) was added to the solution. The mixture was stirred at room temperature for 2 hours. The solvent was removed under reduced pressure. The resulting oily residue was triturated with ethyl acetate (50 ml). The precipitate obtained was stirred under reflux in ethyl acetate (50 ml) for 15 minutes and finally collected by filtration, washed with ethyl acetate and dried; melting point: 246-247 °C; IR: 3349, 2832, 1728, 1670, 1610, 1517, 1403, 1330, 1309, 1260, 1230, 1178 cm⁻¹; ¹H-NMR (DMSO d₆) δ (ppm) 2.50 (m, 4H), 2.80 (m, 4H), 3.10 (m, 4H), 6.85 (d, 2H), 7.35-7.50 (m, 5H), 7.55 (d, 2H), 7.70 (d, 2H), 7.75 (d, 2H), 9.70 (s, 1H), 11.70 (s, 2H), 12.10 (s, I H).

### Example 3: preparation of

### N-{4-[4-(5-(1,1'-Biphenyl)-4-yl-2,4,6-trioxoperhydropyrimidin-5-yl)piperazin-1-yl]-phenylcarbamoyl}butyric acid .

Glutaric anhydride (200 mg) was dissolved in dimethylformamide (5 ml). 5-[4-(4-Aminophenyl)piperazin-1-yl]-5-[1,1'-biphenyl]-4-ylpyrimidine-2,4,6(1*H*,3*H*,5*H*)-trione (500 mg) was added to the solution. The mixture was stirred at room temperature for 2 hours. The solvent was removed under reduced pressure. The resulting oily residue was triturated with ethyl acetate (50 ml).

The precipitate obtained was stirred under reflux in ethyl acetate (50 ml) for 15 minutes and finally collected by filtration, washed with ethyl acetate and dried: melting point: 268-269 °C; IR: 3343, 3191, 3079, 2968, 2836, 1722, 1654, 1603, 1540, 1515, 1335, 1312, 1226 cm⁻¹; ¹H-NMR (DMSO d₆) δ (ppm) 1.80 (m, 2H), 2.25 (m, 4H), 2.80 (m, 4H), 3.10 (m, 4H), 6.85 (d, 2H), 7.35-7.50 (m, 5H), 7.55 (d, 2H), 7.70 (d, 2H), 7.75 (d, 2H), 9.70 (s, 1H), 11.70 (s, 2H), 12.05 (s, 1H).

### Example 4: preparation of

### 5-(1,1'-Biphenyl)-4-yl-5-{4-[4-(2,5-dioxopyrrolidin-1-yl)phenyl]piperazin-1-yl}pyrimidine-2,4,6(1H,3H,5H)-trione

*N*-{4-[4-(5-(1,1'-biphenyl)-4-yl-2,4,6-trioxoperhydropyrimidin-5-yl)piperazin-1-yl]-phenyl}succinamic acid (500 mg) was dissolved in anhydrous tetrahydrofuran (20 ml). 1,1'-Carbonyldiimidazole (250 mg) was added to the solution. The mixture was stirred at room temperature for 2 hours. Triethylamine (0.1ml) was added to the solution. The mixture was stirred at room temperature for 2 hours. The solvent was removed under reduced pressure. Ethyl acetate (20 ml) was added to the residue. A solution of sodium bicarbonate 1% (w/w) was added to the mixture. The mixture was stirred at room temperature for 2 hours. The solid was collected by filtration. The organic layer was dried with anhydrous MgSO₄. Ethyl acetate was removed under reduced pressure. The solids were put together. The compound was stirred in water for 1 hour and then collected by filtration and dried in a vacuum system (containing NaOH pellets) at room temperature: melting point: 310-311 °C; IR: 3202, 3099, 2987, 2849, 1715, 1610, 1450, 1377, 1332, 1315, 1242, 1164 cm⁻¹; ¹H-NMR (DMSO d₆) δ (ppm) 2.75 (s, 4H), 2.80 (m, 4H), 3.20 (m, 4H), 7.00 (d, 2H), 7.05 (d, 2H), 7.40 (t, 1H), 7.50 (t, 2H), 7.55 (d, 2H), 7.70 (d, 2H), 7.75 (d, 2H), 11.70 (s, 2H).

### Example 5: preparation of

### 5-(1,1'-Biphenyl)-4-yl-5-{4-[4-(2,5-dioxopiperidin-1-yl)phenyl]piperazin-1-yl}pyrimidine-2,4,6(1H,3H,5H)-trione.

*N*-{4-[4-(5-(1,1'-Biphenyl)-4-yl-2,4,6-trioxoperhydropyrimidin-5-yl)piperazin-1-yl]-phenyl}phenylcarbamoyl}butyric acid (500 mg) was dissolved in anhydrous tetrahydrofuran (20 ml). 1,1'-Carbonyldiimidazole (250 mg) was added to the solution. The mixture was stirred at room temperature for 2 hours. Triethylamine (0,1ml) was added to the solution. The mixture was stirred at room temperature for 2 hours. The solvent was removed under reduced pressure. Ethyl acetate (20ml) was added to the residue. A solution of sodium bicarbonate 1% (w/w) was added to the mixture. The mixture was stirred at room temperature for 2 hours. The solid was collected by filtration. The organic layer was dried with anhydrous MgSO₄. Ethyl acetate was removed under reduced pressure. The solids were put together. The compound was stirred in water for I hour and then collected by filtration and dried in a vacuum system (containing NaOH pellets) at room temperature: melting point: 300-301 °C; IR: 3212, 2842, 1738, 1698, 1516, 1401, 1373, 1343, 1238, 1177, 1140 cm⁻¹; ¹H-NMR (DMSO d₆) δ (ppm) 1.95 (m, 2H), 2.70 (m, 4H), 2.80 (m, 4H), 3.15 (m, 4H), 6.90 (s, 4H), 7.40 (t, 1H), 7.50 (t, 2H), 7.50 (t, 2H), 7.55 (d, 2H), 7.70 (d, 2H), 7.75 (d, 2H), 11.70 (s, 2H).

### Example 6: preparation of

### N-{4-[4-(5-(1,1'-Biphenyl)-4-yl-2,4,6-trioxoperhydropyrimidin-5-yl)piperazin-1-yl]-phenyl}acetamide

Acetonitrile (1,2 ml) and acetic anhydride (1,2 ml) were added to 5-[1,1'-biphenyl]-4-yl-5-[4-(4-aminophenyl)piperazin-1-yl]pyrimidine-2,4,6(1*H*,3*H*,5*H*)-trione (70 mg). The mixture was stirred at room temperature for 30 minutes. The title compound was precipitated by addition of water (5 ml). The precipitate was collected by filtration and washed with water. The compound was dried in a vacuum system (containing P₂O₅) at room temperature: melting point : 267-268 °C; IR: 3385, 2992, 2832, 1703, 1662, 1600, 1539, 1513, 1413, 1339, 1319, 1222 cm⁻¹.

### Example 7: preparation of

### N-{4-[4-(5-(1,1'-Biphenyl)-4-yl-2,4,6-trioxoperhydropyrimidin-5-yl)-piperazin-1-yl]phenyl}formamide

Formic acid (1 ml) was mixed with acetic anhydride (2 ml). The mixture was stirred at 50°C for 30 minutes (solution A).
Acetonitrile (1 ml) and solution A (1 ml) were added to 5-[1,1'-biphenyl]-4-yl-5-[4-(4-aminophenyl) piperazin-1-yl]pyrimidine-2,4,6(1*H*,3*H*,5*H*)-trione (70 mg). The mixture was stirred at room temperature for 30 minutes. The solvent was evaporated under reduced pressure. The residue was dissolved in hot methanol. The solution was treated with charcoal and then filtered. The product was precipitated by addition of water, collected by filtration and washed with water and dried: melting point: 270-271 °C; IR: 3061, 2836, 1737, 1706, 1518, 1402, 1334, 1314, 1235 cm⁻¹.

## Claims

1. A compound of formula I 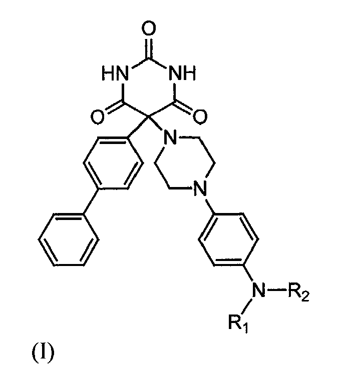 or a pharmaceutical acceptable salt thereof with a pharmaceutically acceptable acid or base, an optical isomer thereof, a tautomeric form thereof, or a polymorphic form thereof wherein :
R₁ is hydrogen ; or R₁ forms with R₂ and the nitrogen atom to which they are attached a succinimide (pyrrolidinedione) cycle, a glutarimide (piperidinedione) cycle or a perhydroazepinedione cycle;
R₂ is formyl; a straight or branched C₁-₆-acyl; a straight or branched carboxy-C₁₋₆-alkylcarbonyl; a straight or branched C₁-₆-alkoxycarbonyl; a straight or branched C₁-₆-alkylaminocarbonyl ;
or R₂ forms with R₁ and the nitrogen atom a succinimide (pyrrolidinedione) cycle, a glutarimide (pyridinedione) cycle or a perhydroazepinedione cycle.

2. A compound according to claim 1 wherein R₁ is Hydrogen and R₂ is carboxy-C₁₋₆ alkylcarbonyl.

3. A compound according to claim 1 selected from the group consisting of:
*N*-{4-[4-(5-(1,1'-biphenyl)-4-yl-2,4,6- trioxoperhydropyrimidin-5-yl)piperazin-1-yl]-phenyl}succinamic acid;
*N*-{4-[4-(5-(1,1'-Biphenyl)-4-yl-2,4,6-trioxoperhydropyrimidin-5-yl)piperazin-1-yl]-phenyl}phenylcarbamoyl}butyric acid;
5-(1,1'-Biphenyl)-4-yl-5-{4-[4-(2,5-dioxopyrrolidin-1-yl)phenyl]piperazin-1-yl}pyrimidine-2,4,6(1*H*,3*H*,5*H*)-trione;
5-(1,1'-Biphenyl)-4-yl-5-{4-[4-(2,5-dioxopiperidin-1-yl)phenyl]piperazin-1-yl}pyrimidine-2,4,6(1*H*,3*H*,5*H*)-trione;
*N*-{4-[4-(5-(1,1'-Biphenyl)-4-yl-2,4,6-trioxoperhydropyrimidin-5-yl)piperazin-1-yl]-phenyl}acetamide;
*N*-{4-[4-(5-(1,1'Biphenyl)-4-yl-2,4,6-trioxoperhydropyrimidin-5-yl)-piperazin-1-yl]phenyl}formamide.

4. A compound according to any one of the preceding claims which acts as inhibitor of metalloproteinases.

5. A pharmaceutical composition comprising a compound according to any one of the preceding claims or a pharmaceutically acceptable salt thereof together with one or more pharmaceutically acceptable carriers or diluents, or any optical isomer or mixture of optical isomers, including racemic mixture or any tautomeric form together with one or more pharmaceutically acceptable carriers or diluents.

6. A pharmaceutical composition for use in the treatment of disease involving metalloproteinases comprising a compound according to any one of the claims I to 4 or a pharmaceutically acceptable salt thereof together with one or more pharmaceutically acceptable carriers or diluents, or any optical isomer or mixture of optical isomers, including racemic mixture or any tautomeric form together with one or more pharmaceutically acceptable carriers or diluents.

7. The pharmaceutical composition according to claim 5 or 6 in the form of an oral dosage unit or parenteral dosage unit.

8. The pharmaceutical composition according to claim 5 to 7 wherein the compound is administered as a dose ranging from 0.05 mg to 1000 mg per day, preferably from 0.1 mg to 500 mg per day, most preferably from 0.1 mg to 200 mg per day.

9. A compound according to any one of the preceding compound claims or a pharmaceutical acceptable salt thereof with a pharmaceutically acceptable acid or base, or any optical isomer or mixture of optical isomers, including racemic mixture or any tautomeric form together with one or more pharmaceutically acceptable acids or bases for therapeutic use.

10. A compound according to any one of the preceding compound claims or a pharmaceutical acceptable salt thereof with a pharmaceutically acceptable acid or base, or any optical isomer or mixture of optical isomers, including racemic mixture or any tautomeric form together with one or more pharmaceutically acceptable acids or bases for therapeutic use in the treatment of diseases involving metalloproteinases.

11. The use of a compound according to any one of the preceding compound claims for preparing a medicament.

12. The use of A compound according to any one of the preceding compound claims or a pharmaceutical acceptable salt thereof with pharmaceutically acceptable carriers or diluents, or any optical isomer or mixture of optical isomers, including racemic mixture or any tautomeric form together with one or more pharmaceutically acceptable carriers or diluents for the preparation of a medicament for the treatment of diseases involving metalloproteinases.

13. A Method of preparing compound according to claim 1 comprising a step of
a) reacting a compound of formula II: 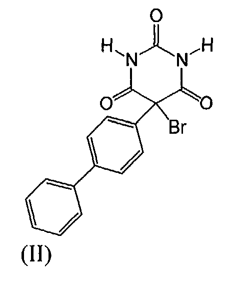 with a compound of formula III: 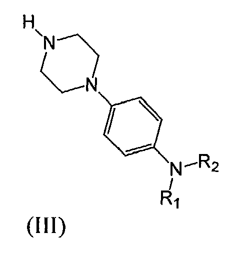
wherein R₁ and R₂ are defined as above, to form a compound of the general formula I.

14. A Method of preparing the compound according to claim 1 comprising a step of
b) reacting a compound of formula VI: 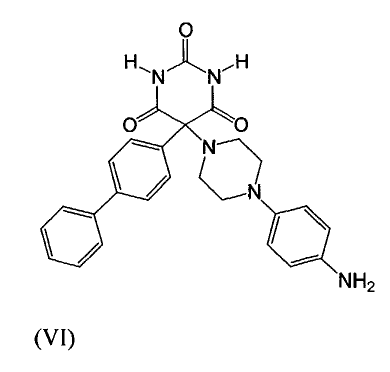 with an appropriate chloroformiate (R'OCOCl), isocyanate (R'NCO) or anhydride selected from the group consisting of (R'CO)₂O, HCOOCOCH₃, succinic anhydride, glutaric anhydride or perhydrooxepine-2,7-dione, wherein R' is a straight or branched C₁₋₆ alkyl chain, optionally followed by a ring closure.

15. A Method of preparing the compound according to claim 1 comprising a step of:
c) reacting a compound of formula (II) 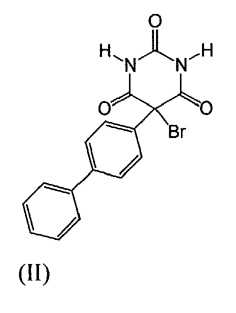 with a compound such as VII, 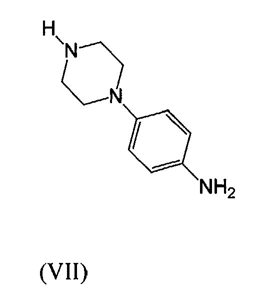 to obtain the compound of formula VI, which is reacted with an appropriate chloroformiate (R'OCOCl), isocyanate (R'NCO) or anhydride selected from the group consisting of (R'CO)₂O, HCOOCOCH₃, succinic anhydride, glutaric anhydride or perhydrooxepine-2,7- dione, wherein R' is a straight or branched C₁₋₆ alkyl chain, optionally followed by a ring closure.
